# EUROPEAN PATENT SPECIFICATION

(11) **EP 2 205 978 B1**
(45) Date of publication and mention of the grant of the patent: **23.11.2011**
(21) Application number: 08832513.9
(22) Date of filing: 16.09.2008
(51) Int. Cl.: G01N 33/68

(54) **ASSESSING CONGESTIVE HEART RISK IN PATIENTS TREATED OR POTENTIALLY TO BE TREATED WITH A PEROXISOME-PROLIFERATOR-ACTIVATOR-RECEPTOR-GAMMA AGONIST OR A THIAZOLIDINEDIONE**
BEURTEILUNG DES RISIKOS VON KONGESTIVER HERZINSUFFIZIENZ BEI PATIENTEN, DIE MIT EINEM PEROXISOM-PROLIFERATOR-AKTIVATOR-REZEPTOR-GAMMA-AGONISTEN ODER EINEM THIAZOLIDINDION BEHANDELT WERDEN ODER MÖGLICHERWEISE DAMIT BEHANDELT WERDEN SOLLEN
ÉVALUATION D'UN RISQUE D'INSUFFISANCE CARDIAQUE CONGESTIVE CHEZ DES PATIENTS TRAITÉS OU POUVANT ÊTRE TRAITÉS AVEC UN AGONISTE DU RÉCEPTEUR GAMMA ACTIVÉ PAR LES PROLIFÉRATEURS DES PEROXYSOMES OU AVEC UN THIAZOLIDINEDIONE

(30) Priority: 17.09.2007 US 973113 P
(43) Date of publication of application: 14.07.2010
(73) Proprietor: BG Medicine, Inc., Waltham, MA 02451 (US)
(72) Inventor: MCBURNEY, Robert, Nicholas, Newton MA 02466 (US); WANG, Shunguang, Carlisle MA 01741 (US); PLASTERER, Tom, Marlboro MA 01752 (US); MUNTENDAM, Pieter, Boxford MA 01921 (US)
(74) Representative: Chapman, Paul William
(86) International application number: PCT/US2008/076492
(87) International publication number: WO 2009/039078

(56) References cited:
- LOK D ET AL: "Abstract 845-8: Galectin-3, a novel marker of macrophage activity, predicts outcome in patients with stable chronic heart failure" JOURNAL OF THE AMERICAN COLLEGE OF CARDIOLOGY, ELSEVIER, NEW YORK, NY, US, vol. 49, no. 9, 27 February 2007 (2007-02-27), pages A98-A98, XP002509330 ISSN: 0735-1097
- GILES T ET AL: "Abstract 1016-64: Preserving Systolic function: two studies demonstrate safety of pioglitazone in patients with type 2 diabetes mellitus and heart failure" JOURNAL OF THE AMERICAN COLLEGE OF CARDIOLOGY, ELSEVIER, NEW YORK, NY, US, vol. 49, no. 9, 27 February 2007 (2007-02-27), pages 66A-66A, XP002509331 ISSN: 0735-1097
- ERDMANN E ET AL: "Abstract 1016-65: Mortality after serious heart failure: results from PROactive" JOURNAL OF THE AMERICAN COLLEGE OF CARDIOLOGY, ELSEVIER, NEW YORK, NY, US, vol. 49, no. 9, 27 February 2007 (2007-02-27), pages 66A-66A, XP002509332 ISSN: 0735-1097
- NESTO RICHARD W ET AL: "Thiazolidinedione use, fluid retention, and congestive heart failure: a consensus statement from the American Heart Association and American Diabetes Association. October 7, 2003." CIRCULATION 9 DEC 2003, vol. 108, no. 23, 9 December 2003 (2003-12-09), pages 2941-2948, XP002509333 ISSN: 1524-4539
- HOTTA K ET AL: "Galectin-12, an Adipose-expressed Galectin-like Molecule Possessing Apoptosis-inducing Activity." THE JOURNAL OF BIOLOGICAL CHEMISTRY 7 SEP 2001, vol. 276, no. 36, 7 September 2001 (2001-09-07), pages 34089-34097, XP002509334 ISSN: 0021-9258

## Description

### FIELD OF THE INVENTION

The present invention relates to methods for assessing congestive heart risk in a patient. More specifically, the present invention provides methods for assessing congestive heart risk in a patient being treated, or eligible to be treated, with a peroxisome-proliferator-activator-receptor-γ (PPAR-γ) agonist or a thiazolidinedione (TZD).

### BACKGROUND

Diabetes mellitus is a major cause of morbidity and mortality. Chronically elevated blood glucose leads to debilitating complications: nephropathy, often necessitating dialysis or renal transplant; peripheral neuropathy; retinopathy leading to blindness; ulceration of the legs and feet, leading to amputation; fatty liver disease, sometimes progressing to cirrhosis; and vulnerability to coronary artery disease and myocardial infarction.

Type 2 diabetes mellitus (T2DM) is the most common form of diabetes accounting for about 90% of all cases of diabetes and affecting 10-20% of those over 45 years of age in many developed countries. It is characterized by defects in insulin action resulting in decreased glucose uptake by muscle and fat and increased hepatic glucose production, and by abnormalities in the normal pattern of glucose-stimulated insulin secretion.

A number of thiazolidinediones (TZDs) have been approved as oral antidiabetic agents. TZDs are selective ligands of the nuclear transcription factor peroxisome-proliferator-activator-receptor-γ (PPAR-γ) which improve glycemic control by increasing insulin sensitivity. Millions of people around the world use medications containing TZDs to treat their Type 2 diabetes. However, a significant risk of congestive heart failure has been associated with the use of medications containing TZDs. Continued post-marketing reports of heart failure have prompted the US Food and Drug Administration (FDA) to increase the prominence of this safety concern in the labels for drugs containing TZDs. (FDA Alert 8/2007 (pioglitazone HCl) and FDA Alert 8/14/2007 (rosiglitazone); U.S. FDA website).
Lok et al, Journal of the American College of Cardiology, 49, No. 9 (2007), pA98, discloses that galectin-3 is a maker which predicts mortality in stable chronic heart failure patients.
Giles et al, Journal of the American College of Cardiology, 49, No. 9 (2007), p66A describes thiazolidinediones as being safe insulin-sensitizing agents usable to treat type 2 diabetes patients having heart failure.
Friedmann et al, Journal of the American College of Cardiology, 49, No. 9 (2007), p66A discloses that pioglitazone reduced major cardiovascular events in patients with type 2 diabetes and significant cardiovascular disease who are at risk of heart failure.
Nesto Richard et al, Circulation, 108, No. 23 (2003), p2941-2948, discloses thiazolidinedione having a beneficial effect on cardiovascular risk factors and therefore being attractive agents in patients with type 2 diabetes who are at high risk for cardiovascular disease, such as congestive heart failure.
Hotta et al, The Journal of Biological Chemistry, 276, No. 36 (2001), p34089-34097, discloses that gatectin-12 expression is induced by thiazolidinediones.

Because of the importance of PPAR-γ agonists and TZDs as antidiabetic agents, there remains a need to develop methods for effectively diagnosing heart failure in patients who rely on these medications.

### SUMMARY OF THE INVENTION

The present invention provides methods for assessing a risk of congestive heart failure in a patient being treated with a peroxisome-proliferator-activator-receptor-γ (PPAR-γ) agonist or a thiazolidinedione (TZD) by detecting or monitoring the concentration of one or more markers associated with heart failure. As a result, the present invention allows patients to more safely use PPAR-γ agonists and thiazolidinedione compounds as therapeutic agents.
Accordingly, one aspect of the present invention relates to a method of assessing congestive heart failure risk in a patient being administered thiazolidinedione, the method comprising:
detecting the presence or absence of an increasing galectin-3 concentration in a body fluid sample of a patient being treated with thiazolidinedione,
wherein the presence of an increasing galectin-3 concentration over time is indicative of an increased congestive heart failure risk in the patient.
In another aspect the present invention relates to a method of assessing a candidate for treatment with a thiazolidinedione, the method comprising:
measuring a galectin-3 concentration in a body fluid sample of a patient having a condition treatable with a thiazolidinedione;
comparing the measured galectin-3 concentration to a reference galectin-3 concentration, wherein the reference galectin-3 concentration is derived from concentrations of galectin-3 in other patients having the condition and is indicative of congestive heart failure risk in patients having the condition; and
recommending that administration of the thiazolidinedione be restricted or discontinued if the measured galectin-3 concentration exceeds the reference galectin-3 concentration. The result of the comparison is used to assess whether the measured galectin-3 concentration is indicative of an increased congestive heart risk in the patient.
The invention includes assessing a patient (*e.g.*, a diabetes patient) which includes measuring a change over time in galectin-3 concentration in a body fluid (*e.g.,* blood, serum, or plasma) of the patient being treated with a thiazolidinedione (*e.g.*, rosiglitazone), and comparing the measured change to changes in galectin-3 concentration observed in other patients treated with the thiazolidinedione for whom congestive heart status is known or determinable. The result of the comparison is used to assess whether the measured change in galectin-3 concentration is indicative of an increased congestive heart risk in the patient. The method can include comparing a galectin-3 concentration during a course of treatment with the thiazolidinedione to an earlier galectin-3 concentration during the course of treatment. The method can also include comparing galectin-3 levels measured at several times during the course of treatment to develop a treatment history of galectin-3 concentrations.
The invention also includes assessing the patient by detecting the presence or absence of an increasing galectin-3 concentration in a body fluid (*e.g.*, blood, serum, or plasma) of a patient being treated with a thiazolidinedione (*e.g.*, rosiglitazone). The presence of an increasing galectin-3 concentration over time is indicative of an increased congestive heart risk in the patient. The method can include comparing a galectin-3 concentration during a course of treatment with the thiazolidinedione to an earlier galectin-3 concentration during the course of treatment. The method can also include comparing galectin-3 levels at several times during the course of treatment to develop a treatment history of gatectin-3 concentrations.

The results of the risk assessment can be used to inform future decisions involving treatment of the patient. For example, if a patient appears to have an increased congestive heart risk, based on the patient's galectin-3 concentration or a change in the patient's galectin-3 concentration, administration of the thiazolidinedione may be discontinued, limited, or restricted, such as by reducing the dose or frequency of administration. The patient may be switched to a different medication, such as a different thiazolidinedione or a non-thiazolidinedione such as glyburide or metformin.
Alternatively, if the patient does not appear to have a substantially increased congestive heart risk, administration of the thiazolidinedione may continue, be extended, or be increased (*e.g.* in dose or frequency, if permissible dose or frequency have not yet been reached).

Similarly, the invention provides a method for assessing a candidate (*e.g.*, a diabetes patient) for treatment with a thiazolidinedione (*e.g.*, rosiglitazone). The method includes measuring a galectin-3 concentration in a body fluid of a patient having a condition treatable with a thiazolidinedione and comparing the measured galectin-3 concentration to a reference galectin-3 concentration. The reference galectin-3 concentration can be derived from observed concentrations of galectin-3 in other patients having the condition, and is indicative of congestive heart risk in patients having the condition. Thiazolidinedione treatment can be restricted or refused if the measured galectin-3 concentration exceeds the reference galectin-3 concentration.
In another aspect the invention relates to a method of assessing congestive heart failure risk in a patient being administered a peroxisome-proliferator-activator-receptor-γ agonist, the method comprising:
detecting the presence or absence of an increasing galectin-3 concentration in a body fluid sample of a patient being treated with a peroxisome-proliferator-activator-receptor-γ agonist,
wherein the presence of an increasing galectin-3 concentration over time is indicative of an increased congestive heart failure risk in the patient.
The invention includes assessing a patient (*e.g*, a diabetes patient) which includes measuring a change over time in galectin-3 concentration in a body fluid (*e.g.*, blood, serum, or plasma) of the patient being treated with a PPAR-γ agonist, and comparing the measured change to changes in galectin-3 concentration observed in other patients treated with the PPAR-γ agonist for whorn congestive heart status is known or determinable. The result of the comparison is used to assess whether the measured change in galectin-3 concentration is indicative of an increased congestive heart risk in the patient. The method can include comparing a galectin-3 concentration during a course of treatment with the PPAR-γ agonist to an earlier galectin-3 concentration during the course of treatment. The method can also include comparing galectin-3 levels measured at several times during the course of treatment to develop a treatment history of galectin-3 concentrations.
Preferably, the peroxisome-proliferator-activator-receptor- y agonist is a thiazolidinedione.
The invention includes assessing the patient by detecting the presence or absence of an increasing galectin-3 concentration in a body fluid (*e.g.*, blood, serum, or plasma) of a patient being treated with a PPAR-γ agonist. The presence of an increasing galectin-3 concentration over time is indicative of an increased congestive heart risk in the patient. The method can include comparing a galectin-3 concentration during a course of treatment with the PPAR-γ agonist to an earlier galectin-3 concentration during the course of treatment. The method can also include comparing galectin-3 levels at several times during the course of treatment to develop a treatment history of galectin-3 concentrations.

The results of the risk assessment can be used to inform future decisions involving treatment of the patient. For example, if a patient appears to have an increased congestive heart risk, based on the patient's galectin-3 concentration or a change in the patient's galectin-3 concentration, administration of the PPAR-γ agonist may be discontinued, limited, or restricted, such as by reducing the dose or frequency of administration. The patient may be switched to a different medication, such as a different PPAR-γ agonisst. Alternatively, if the patient does not appear to have a substantially increased congestive heart risk, administration of the PPAR-γ agonist may continue, be extended, or be increased (*e.g*. in dose or frequency, if permissible dose or frequency have not yet been reached).

Similarly, the invention provides a method for assessing a candidate (*e.g*., a diabetes patient) for treatment with a PPAR-γ agonist. The method includes measuring a galectin-3 concentration in a body fluid of a patient having a condition treatable with a PPAR-γ agonist and comparing the measured galectin-3 concentration to a reference galcctin-3 concentration. The reference galectin-3 concentration can be derived from observed concentrations of galectin-3 in other patients having the condition, and is indicative of congestive heart risk in patients having the condition. PPAR-γ agonist treatment can be restricted or refused if the measured galectin-3 concentration exceeds the reference galectin-3 concentration.

### DETAILED DESCRIPTION OF THE INVENTION

Applicants have invented a method of assessing congestive heart risks in patients treated with a peroxisome-proliferator-activator-receptor-γ (PPAR-γ) agonist or a thiazolidinedione (TZD). TZD-based drugs are used to treat type II diabetes and other diseases, such as liposarcoma and polycystic ovary disease. Recently, clinical studies have suggested that TZD administration may cause or worsen heart failure in certain patients. By assessing an individual patient's risk of developing or aggravating a heart condition, appropriate decisions regarding that patient's treatment options can be made.

Applicants have developed methods permitting the use of circulating galectin-3 protein levels as an indicator for congestive heart risks in patients being treated, or patients eligible to be treated, with a PPAR-γ agonist or a TZD. Elevated concentrations of galectin-3 in body fluids, when compared to galectin-3 levels observed in healthy individuals, have been correlated with the development of CHF (van Kimmenade et al., J. Am. Coll. Cardiol., 48:1217-24 (2006)). Applicants' research has revealed, however, that the comparing the galectin-3 levels in a diabetes patient being treated with a PPAR-γ agonist or a TZD to galectin-3 levels observed in healthy individuals is unlikely to be informative. First, Applicants have discovered that diabetes itself leads to a significant increase in galectin-3 expression. If galectin-3 levels are frequently elevated in diabetes patients as a result of the diabetes, detecting an elevated galectin-3 level in a diabetes patient compared to a healthy individual would be unsurprising and not necessarily informative of the condition of the patient's heart. Second, Applicants have also discovered that administration of a PPAR-γ agonist or a TZD can reduce galectin-3 expression. In combination, the diabetes-dependent increase in galectin-3 expression and the PPAR-γ agonist- or TZD-dependent decrease in galectin-3 expression would complicate an effort to draw meaningful conclusions based on comparisons to healthy individuals.

Having discovered these competing effects on galectin-3 expression, Applicants have developed new methods for using galectin-3 levels as an indicator of congestive heart risk. Whereas a comparison of galectin-3 levels to those in healthy patients may be uninformative, a comparison of galectin-3 levels to those in comparable patients (e.g. patients with the same disease or condition, perhaps treated with the same PPAR-γ agonist or TZD) can reveal important information about a patient's heart status or risk.

Thus, in some embodiments, a patient taking a PPAR-γ agonist or a TZD is assessed for a risk of congestive heart by measuring the level of galectin-3 (and/or other markers of heart failure) in the patient and comparing the measured galectin-3 concentration to a galectin-3 concentration which has been detected in other patients being treated with the PPAR-γ agonist or TZD for whom the congestive heart status is known. The other patients may be matched for age, gender, or the diseases being treated. Galectin-3 concentrations in the patient being treated can also be measured over time and compared to temporal changes in galectin-3 concentration observed in other, similarly-treated patients whose congestive heart status is known. Changes in galectin-3 levels resulting from heart failure can thereby be distinguished from the presence of a particular disease (e.g., diabetes) or from changes in galectin-3 levels resulting from treatment with the PPAR-γ agonist or the TZD. Thus, if the patient being treated has galectin-3 levels or changes in galectin-3 levels similar to other patients who are known to have a congestive heart, then the patient being treated may be at risk for congestive heart failure.

Alternatively, a galectin-3 concentration can be measured in a patient taking a PPAR-γ agonist or a TZD and the galectin-3 concentration can be compared to a previous galectin-3 concentration measured in the patient. An increase in galectin-3 concentration relative to one or more previous galectin-3 concentrations in the patient is an indication that the patient is at risk for a congestive heart. Marker levels can be monitored over time, such as in samples obtained from a patient at annual, semi-annual, bimonthly, monthly, triweekly, biweekly, weekly, daily, or at variable intervals.

Treatment with a PPAR-γ agonist or a TZD may be modified or terminated if the patient is determined to be at and increased risk for a congestive heart. For example, the PPAR-γ agonist or TZD dosage amount may be decreased or the frequency of administration may be reduced until the risk for congestive heart failure is reduced to an acceptable level.

Multimarker analysis can be used to improve the accuracy of diagnosis and monitoring. For example, blood concentrations of galectin-3 (Gal-3) and brain natriuretic peptide (BNP) can be used to diagnose heart failure and to predict the long-term outcome of heart failure (van Kimmenade et al., J. Am. Coll. Cardiol., 48:1217-24 (2006); Sharma et al., Circulation, 110:3121-28 (2004); Lok et al., Eur. Heart J., 28:141, Abstract 1035 (2007)). BNP and its cleavage equivalent amino-terminal proBNP (NT-proBNP) are elevated in heart muscle and in blood during heart failure as a result of high filling pressures of heart chambers and the stretch of cardiac muscle fibers. Other secondary markers that could be used to diagnose heart failure may include non-polypeptidic cardiac markers such as sphingolipid, sphingosine, sphingosine-1-phosphate, dihydrosphingosine and sphingosylphosphorylcholine (see U.S. Pat. No. 6,534,322). When measuring the levels of the above markers, corrections for age and gender may be incorporated to improve the accuracy of diagnosis.

### Marker Detection:

The present invention provides methods for detecting and monitoring heart failure in patients taking medications containing a PPAR-γ agonist or a TZD by measuring the levels of one or more markers (e.g., Gal-3, BNP, NT-proBNP). Many methods for detecting of a protein of interest, with or without quantitation, are well known and can be used in the practice of the present invention. Examples of such assays are described below and can include, for example, immunoassays, chromatographic methods, and mass spectroscopy. Such assays can be performed on any biological sample including, among others, blood, plasma, and serum. Accordingly, multiple assays can be used to detect Gal-3 or BNP, and samples can be analyzed from one or more sources.

Markers can be detected or quantified in a sample with the help of one or more separation methods. For example, suitable separation methods may include a mass spectrometry method, such as electrospray ionization mass spectrometry (ESI-MS), ESI-MS/MS, ESI-MS/(MS)ⁿ (n is an integer greater than zero), matrix-assisted laser desorption ionization time-of-flight mass spectrometry (MALDI-TOF-MS), surface-enhanced laser desorption/ionization time-of-flight mass spectrometry (SELDI-TOF-MS), desorption/ionization on silicon (DIOS), secondary ion mass spectrometry (SIMS), quadrupole time-of-flight (Q-TOF), atmospheric pressure chemical ionization mass spectrometry (APCI-MS), APCI-MS/MS, APCI-(MS)ⁿ, or atmospheric pressure photoionization mass spectrometry (APPI-MS), APPI-MS/MS, and APPI-(MS)ⁿ. Other mass spectrometry methods may include, inter alia, quadrupole, fourier transform mass spectrometry (FTMS) and ion trap. Spectrometric techniques that can also be used include resonance spectroscopy and optical spectroscopy.

Other suitable separation methods include chemical extraction partitioning, column chromatography, ion exchange chromatography, hydrophobic (reverse phase) liquid chromatography, isoelectric focusing, one-dimensional polyacrylamide gel electrophoresis (PAGE), two-dimensional polyacrylamide gel electrophoresis (2D-PAGE), or other chromatographic techniques, such as thin-layer, gas or liquid chromatography, or any combination thereof. In one embodiment, the biological sample to be assayed may be fractionated prior to application of the separation method.

Markers may be detected or quantified by methods that do not require physical separation of the markers themselves. For example, nuclear magnetic resonance (NMR) spectroscopy may be used to resolve a profile of a marker from a complex mixture of molecules. An analogous use of NMR to classify tumors is disclosed in Hagberg, NMR Biomed. 11: 148-56 (1998), for example.

A marker in a sample also may be detected or quantified, for example, by combining the marker with a binding moiety capable of specifically binding the marker. The binding moiety may include, for example, a member of a ligand-receptor pair, i.e., a pair of molecules capable of having a specific binding interaction. The binding moiety may also include, for example, a member of a specific binding pair, such as antibody-antigen, enzyme-substrate, nucleic acid-nucleic acid, protein-nucleic acid, protein-protein, or other specific binding pairs known in the art. Binding proteins may be designed which have enhanced affinity for a target. Optionally, the binding moiety may be linked with a detectable label, such as an enzymatic, fluorescent, radioactive, phosphorescent or colored particle label. The labeled complex may be detected, e.g., visually or with the aid of a spectrophotometer or other detector, or may be quantified.

The markers measured in the present invention may be detected using an immunoassay. For example, an enzyme-linked immunosorbant assay kit (ELISA) for detecting Gal-3 is commercially available (Bender Medsystems, Vienna, Austria). In addition, antibodies binding to BNP can be obtained commercially. Examples of commercially available antibodies binding to BNP are rabbit anti-human BNP polyclonal antibody (Biodesign International), rabbit anti-BNP amino acids 1-20 polyclonal antibody (Biodesign International), anti-human BNP monoclonal antibody (Immundiagnostik), and rabbit anti-human BNP amino acids 1-10 polyclonal antibody (Immundiagnostik).

An immunoassay can be performed by contacting a sample from a subject to be tested with an appropriate antibody under conditions such that immunospecific binding can occur if the marker is present, and detecting or measuring the amount of any immunospecific binding by the antibody. Any suitable immunoassay can be used, including, without limitation, competitive and non-competitive assay systems using techniques such as Western blots, radioimmunoassays, ELISA (enzyme linked immunosorbent assay), "sandwich" immunoassays, immunoprecipitation assays, immunodiffusion assays, agglutination assays, complement-fixation assays, immunoradiometric assays, fluorescent immunoassays and protein A immunoassays.

In a sandwich immunoassay, two antibodies capable of binding a marker generally are used, *e.g.*, one immobilized onto a solid support, and one free in solution and labeled with a detectable chemical compound. Examples of chemical labels that may be used for the second antibody include radioisotopes, fluorescent compounds, and enzymes or other molecules that generate colored or electrochemically active products when exposed to a reactant or enzyme substrate. When a sample containing the marker is placed in this system, the marker binds to both the immobilized antibody and the labeled antibody, to form a "sandwich" immune complex on the support's surface. The complexed marker is detected by washing away non-bound sample components and excess labeled antibody, and measuring the amount of labeled antibody complexed to the marker on the support's surface. Alternatively, the antibody free in solution, which can be labeled with a chemical moiety, for example, a hapten, may be detected by a third antibody labeled with a detectable moiety which binds the free antibody or, for example, the hapten coupled thereto.

Both the sandwich immunoassay and tissue immunohistochemical procedures are highly specific and very sensitive, provided that labels with good limits of detection are used. A detailed review of immunological assay design, theory and protocols can be found in numerous texts in the art, including Butt, W.R., Practical Immunology, ed. Marcel Dekker, New York (1984) and Harlow et al. Antibodies, A Laboratory Approach, ed. Cold Spring Harbor Laboratory (1988).

In general, immunoassay design considerations include preparation of antibodies (*e.g.*, monoclonal or polyclonal antibodies) having sufficiently high binding specificity for the target to form a complex that can be distinguished reliably from products of nonspecific interactions. As used herein, the term "antibody" is understood to mean binding proteins, for example, antibodies or other proteins comprising an immunoglobulin variable region-like binding domain, having the appropriate binding affinities and specificities for the target. The higher the antibody binding specificity, the lower the target concentration that can be detected. As used herein, the terms "specific binding" or "binding specifically" are understood to mean that the binding moiety, for example, a binding protein, has a binding affinity for the target of greater than about 10⁵ M⁻¹, more preferably greater than about 10⁷ M⁻¹.

Antibodies to an isolated target marker which are useful in assays for detecting heart failure in an individual may be generated using standard immunological procedures well known and described in the art. See, for example Practical Immunology, *supra*. Briefly, an isolated marker is used to raise antibodies in a xenogeneic host, such as a mouse, goat or other suitable mammal. The marker is combined with a suitable adjuvant capable of enhancing antibody production in the host, and is injected into the host, for example, by intraperitoneal administration. Any adjuvant suitable for stimulating the host's immune response may be used. A commonly used adjuvant is Freund's complete adjuvant (an emulsion comprising killed and dried microbial cells and available from, for example, Calbiochem Corp., San Diego, or Gibco, Grand Island, NY). Where multiple antigen injections are desired, the subsequent injections may comprise the antigen in combination with an incomplete adjuvant (e.g., cell-free emulsion). Polyclonal antibodies may be isolated from the antibody-producing host by extracting serum containing antibodies to the protein of interest. Monoclonal antibodies may be produced by isolating host cells that produce the desired antibody, fusing these cells with myeloma cells using standard procedures known in the immunology art, and screening for hybrid cells (hybridomas) that react specifically with the target and have the desired binding affinity.

Antibody binding domains also may be produced biosynthetically and the amino acid sequence of the binding domain manipulated to enhance binding affinity with a preferred epitope on the target. Specific antibody methodologies are well understood and described in the literature. A more detailed description of their preparation can be found, for example, in Practical Immunology, *(supra).*

In addition, genetically engineered biosynthetic antibody binding sites, also known in the art as BABS or sFv's, may be used to determine if a sample contains a marker. Methods for making and using BABS comprising (i) non-covalently associated or disulfide bonded synthetic V_{H} and V_{L} dimers, (ii) covalently linked V_{H}-V_{L} single chain binding sites, (iii) individual V_{H} or V_{L} domains, or (iv) single chain antibody binding sites are disclosed, for example, in U.S. Patent Nos.: 5,091,513; 5,132,405; 4,704,692; and 4,946,778. Furthermore, BABS having requisite specificity for the marker can be derived by phage antibody cloning from combinatorial gene libraries (see, for example, Clackson et al. Nature 352: 624-628 (1991)). Briefly, phages, each expressing on their coat surfaces BABS having immunoglobulin variable regions encoded by variable region gene sequences derived from mice pre-immunized with an isolated marker, or a fragment thereof, are screened for binding activity against the immobilized marker. Phages which bind to the immobilized marker are harvested and the gene encoding the BABS is sequenced. The resulting nucleic acid sequences encoding the BABS of interest then may be expressed in conventional expression systems to produce the BABS protein.

### EXAMPLES

### Example 1: Animal Models of Diabetes and Administration of PPAR-γ agonists and TZDs

The overall experimental design was developed to characterize biomarker fingerprints for three medicines used in the treatment of Type 2 Diabetes Mellitus (T2DM) - rosiglitazone maleate (Rosi), glyburide (Gly) and metformin (Met). In order to accomplish this goal, drug response was assessed in two experimental mouse models of T2DM.

One mouse model was the diet-induced obesity model (HFD) in which C57BL/6 mice were placed on a high fat dict. This strain of mice is genetically susceptible to diet-induced obesity, hyperglycemia, and hyperinsulinemia (Surwit et al. Diabetes. 37:1163-7 (1988)). When placed on a high-fat, high-simple-carbohydrate diet (58% kcal fat) for 8-12 weeks, these animals develop obesity, mild hyperglycemia, and hyperinsulinemia. These mice also develop a phenotype characteristic of dyslipidemia, including elevated cholesterol, triglycerides, and free-fatty acids.

The other mouse model was the db/db mouse model (DB) of T2DM. The db/db mouse is a widely-used genetic model of T2DM characterized by moderate to severe hyperglycemia and hyperinsulinemia (Hummel et al. Science. 153:1127-8 (1966)). These mice have a spontaneous mutation in the leptin receptor, *lepr,* and defective leptin-mediated signal transduction, which results in chronic overeating. Elevated plasma insulin appears between 10-14 days, obesity is notable at 3-4 weeks of age, and hyperglycemia is apparent at 4-8 weeks of age. Serum triglycerides (TG) and free fatty acids (FFA) are also elevated. db/db mice on the C57BL background were used.

Wildtype C57BL/6 mice were used as non-diabetic controls for the HFD mice. db/+ mice were used as non-diabetic controls for the DB mice. The control mice received the same doses of study drug as the disease model mice. Male mice were used for all studies.

### Sample Size Considerations:

In pilot studies, sample size calculations for the transcriptomics platform were carried out in db/db mice. In order to assess the power to detect differential expression among biologically relevant genes, a list of 78 metabolic related genes for T2DM was compiled from the literature. Analysis of liver tissue for db/+ and db/db animals demonstrated that using a type I error of 0.05, fold change of 2, and power of 0.8, 10 animals per group was sufficient to identify 61.5% of the 78 selected transcripts as differentially expressed. From this analysis, it was determined that 10 animals per treatment group would adequately identify biomarkers of interest.

### Animal Protocol:

### Studies DB06 and DB07

Male db/db and db/+ mice were ordered from The Jackson Laboratory (Bar Harbor, Maine) and arrived at 4-wks of age. The mice were housed in groups of five on a 12:12-hour light-dark cycle and at 23 ± 2°C. After a 1-week quarantine, mice were acclimated to the study conditions for 3-wks. Mice were maintained on a standard rodent chow (Purina 5001; TestDiet, Richmond, IN). All mice had free access to water throughout the experiment. At 7 weeks of age, a blood sample was collected and mice were assigned to one of the treatment groups based on mean plasma glucose levels. Mice were orally dosed with vehicle (0.5% methylcellulose) or Rosi (10 mg/kg) in vehicle or Gly (10mg/kg) in vehicle or Met (75mg/kg) in vehicle for 14 days beginning at 8 weeks of age. All treatments were given via oral gavage (10 ml/kg) and dosing was performed twice/day at approximately 8 a.m. and 3 p.m.

During the treatment period, body weight was measured daily in the morning prior to dosing. Food intake was measured weekly beginning one week prior to the start of the study. Food intake was assessed by giving a pre-weighed amount of food at the beginning of each week and subtracting the food remaining at the end of the week.

On Day 14, mice were euthanized by cervical dislocation. Tissues (liver, inguinal subcutaneous fat and gastrocnemius muscle) were harvested and prepared for histology and transcriptome analysis. Liver, subcutaneous fat, heart, and kidney weights were recorded. For histological analysis, a small section of each tissue was collected in 10% formalin and transferred to 70% ethanol after 24-hr. Briefly, for transcriptome analysis, each tissue was minced in 2.0 to 5.0 ml of RNALater® (Ambion, Inc., Austin, TX) and frozen on dry ice and transferred to longer term storage at -80°C.

### Studies HFD06 and HFD07

Male C57BL/6 mice were ordered from The Jackson Laboratory (Bar Harbor, Maine) and arrived at 4-wks of age. The mice were housed in groups of five on a 12:12-hour (h) light-dark cycle and at 23 ± 2°C. After a one week quarantine, mice were placed on either a 58% HFD (D12331; Research Diets Inc., New Brunswick, NJ) or an 11% standard fat diet (D12329). Mice were maintained on their respective diets for the duration of the experiments. All mice had free access to water throughout the experiment.

After 7 weeks on diet, a blood sample was collected and mice were assigned to one of four treatment groups based on mean plasma glucose levels. Mice were orally dosed with vehicle (0.5% methylcellulose) or Rosi (10 mg/kg) in vehicle or Gly (10mg/kg) in vehicle or Met (75mg/kg) in vehicle for 14-days beginning at 8 weeks of age. All treatments were given via oral gavage (10 ml/kg) and dosing was performed twice/day at approximately 8 a.m. and 3 p.m. All treatments were given via oral gavage (10 ml/kg) and dosing was performed twice/day at approximately 8 a.m. and 3 p.m.

During the treatment period, body weight was measured daily in the morning prior to dosing. Food intake was measured weekly beginning one week prior to the start of the study. Food intake was assessed by giving a pre-weighed amount of food at the beginning of each week and subtracting the food remaining at the end of the week.

On day 14, mice were euthanized by cervical dislocation. Tissues (liver, inguinal subcutaneous fat and gastrocnemius muscle) were harvested and prepared for histology and transcriptome analysis. Liver, subcutaneous fat, heart and kidney weights were recorded. For histological analysis, a small section of each tissue was collected in 10% formalin and transferred to 70% ethanol after 24-hr. Briefly, for transcriptome analysis, each tissue was minced in 2.0 to 5.0 ml of PNALater® (Ambion, Inc., Austin, TX) and frozen on dry ice and transferred to longer term storage at -80°C.

### Health and Safety Assessments:

The general health of the mice on study was monitored daily by research staff. Health assessments included visual observations and daily body weight measurements. If at any time during study, mice exhibited signs of distress or sickness behavior, such as severe lethargy, hunched posture, stereotypic movements, abnormal breathing, or extreme weight loss, they were promptly euthanized via carbon dioxide (CO₂) inhalation.

### Example 2: Transcriptional Profiling of Gal-3 and BNP in mouse T2DM models

Transcriptional analysis (TA) of genes and Expressed Sequence Tags (EST) provides valuable information about biological processes. Affymetrix GeneChip^{®} Technology (Affymetrix, Santa Clara, CA) can be used to analyze global changes in gene expression. In brief, this technology uses messenger ribonucleic acid (mRNA) from an experimental condition to obtain complementary deoxynucleic acid (cDNA), and ultimately, complementary ribonucleic acid (cRNA) for hybridization to GeneChip^{®} arrays. GeneChips^{®} contain nucleic acid probes for thousands of sequences that are bound to a solid surface. Affymetrix GeneChip^{®} technology was used to assay transcriptional changes in three tissues (liver, subcutaneous fat, gastrocnemius muscle) in the preclinical animal studies in Example 1. Samples were hybridized to the GeneChip^{®} Mouse Genome 430A Array. Relative mRNA intensity levels for > 22,000 probe sets were obtained using Affymetrix Microarray Suite^{®} version 5.0 (MAS 5.0, Affymetrix Microarray Suite, Santa Clara, CA).

Tissues collected for transcriptional analysis included liver, subcutaneous fat and gastrocnemius muscle at Day 14 of the preclinical animal studies in Example 1.

### Sample Preparation:

At the time of dissection, a lobe of liver (100 to 200 mg), a section of subcutaneous fat (100 to 350 mg) and one gastrocnemius muscle (100 to 200 mg) were harvested, minced finely (1 to 3 mm) and placed into 5 to 10 volumes of RNAlater® (Ambion, Inc., Austin, TX). RNAlater®, an ammonium sulfate solution, was used to prevent degradation of RNA during the experimental procedures. Samples were stored on dry ice and transferred to a -80°C freezer until further processing.

### RNA Extraction:

Tissue from RNAlater® stocks was weighed, transferred to Trizol reagent (Invitrogen, Carlsbad, CA), and homogenized using the MixAMil system (Retsch, Haan, Germany). RNase-free water, chloroform, and the Trizol-tissue homogenate was spun in a Phase Lock Gel^{™} (PLG) tube (VWR International, West Chester, PA). Clear aqueous supernatant was recovered from the top layer of the PLG, transferred to RNeasy® Mini columns (Qiagen Inc., Valencia, CA) and processed according to manufacturer's instructions. RNA samples were DNAse I treated as recommended. RNA integrity was assessed by Optical Density (OD) ratios (Spetramax, Molecular Devices Corp., Sunnyvale, CA) and ribosomal quality as measured by the Agilent BioAnalyzer^{™} RNA chips and software (Agilent Technologies Inc., Palo Alto, CA).

### Sample Labeling:

Five micrograms of mRNA was used for each sample. cDNA synthesis (Invitrogen Carlsbad, CA) and *in vitro* transcription incorporating biotinylated nucleotides (Enzo Biochem Inc., Farmingdale, NY) was carried out according to standard operating procedures recommended by Affymetrix. Labeling quality was assessed by cRNA yields and integrity as monitored by Agilent BioAnalyzer RNA chips and software.

### Data Acquisition:

Hybridization cocktails containing 10 µg of representative sample cRNA were loaded onto GeneChip^{®} Mouse Genome 430A Array and hybridized overnight. Genechips^{®} were washed and scanned using Affymetrix^{®} fluidic stations and scanners. Intensity data were captured by Genechip^{®} Computer Operating System (GCOS) using the algorithm, MAS 5.0.

### Data Processing and Quality Control:

An initial visual inspection of each chip was completed that checked for uniform color, unexpected spots or scratches, and proper grid alignment. Technical quality control (QC) of all microarray data was performed using the MAS 5.0 analysis software. In addition, S-Plus® (Insightful Corp., Seattle, WA) and Simca-P^{™} software (Umetrics, Umeå, Sweden) were used to perform correlation calculations and provide a general overview of the data.

For all transcriptomics data, the processed data were log-transformed (base 10) before data analysis.

The results reported in Tables 1 & 2 are from a specific focus on the changes in the mRNAs for Galectin-3 (Gal-3) and Brain Natriuretic Peptide (BNP).

### Data Analysis Methods:

The overall data analysis strategy undertaken in this study included: a) performing univariate data analysis (UVDA), which include standard statistical approaches such as analysis of variance (ANOVA) applied to individual analytes separately; b) performing MVDA, which employs a single statistical analysis that applies to individual analytes jointly; c) performing correlation analyses to identify biomarkers that correlate to primary endpoints, such as glucose, insulin, and HbA1c; d) testing different pathway analysis tools to try and combine individual results into a more comprehensive biological pathway understanding; and, e) performing various within sample/tissue integrated statistical analyses to identify common fingerprints across platforms and samples. In general all of these approaches were utilized within each of the platform datasets; however, because of the slight differences in platform data, for example, better annotation of some platform data than others, not all analyses were conducted on each platform.

In the sections below, the general considerations for data analysis relevant to the current study are described, followed by details for each of the statistical and analysis approaches utilized.

### Analysis Population:

The "Analysis Population" consisted of all mice that were not excluded due to the reasons listed below and had data from one or more platforms. The "Analysis Population" was the only population used in the analysis of the data. A mouse was removed from the study due to the death of a mouse, a sharp decrease in body weight, or a moribund appearance.

### Treatment Comparisons:

Comparisons between the mice on each drug and the mice on vehicle, or between the db/db and the db/+ mice or between the HFD and non-HFD mice were made in order to identify significant differences related to drug action and disease, respectively.

### Disease Biomarkers:

Disease biomarkers are related to the disease phenotype (e.g., db/db-specific), and were developed by comparison of the db/db mice treated with vehicle to the db/+ mice treated with vehicle or the HFD mice treated with vehicle to the non-HFD mice treated with vehicle.

### Drug Effect Biomarkers:

db/db-specific drug biomarkcrs were defined as those that were significantly changed by drug treatment and were developed by comparison of the db/db mice treated with drug to the db/db mice treated with vehicle.

HFD-specific drug biomarkers were defined as those that were significantly changed by drug treatment and were developed by comparison of the HFD mice treated with drug to the non-HFD mice treated with vehicle.

### Disease and Drug Biomarkers:

Disease and drug biomarkers were defined as those that were significantly affected by both drug and disease and were called "disease and drug biomarkers."

Drug-by-disease biomarkers were defined as those biomarkers which exhibited a significant drug-x-disease interaction and were called "drug-by-disease biomarkers."

### Outlier Detection:

In this study, a statistical outlier detection method was utilized for transcriptomic platform data in an attempt to ensure high-integrity data. A statistical approach was necessary because several thousand data points were generated in the transcriptomic platform and it was impossible to visually inspect all data points for errors. Thus, a conservative approach was utilized to remove extreme outliers that could negatively affect the ability to detect statistical significance. In this study, univariate outlier detection methods were utilized to identify extreme outliers.

### Univariate Outlier Detection:

Box plots were used to define extreme outliers in the study groups to be excluded from the analysis. Cohorts were analyzed separately for outlier detection. Any data points that lay 2 times the inter-quartile-ranges either below the 25th percentile or above the 75th percentile were deemed extreme outliers.

### Missing Data:

The missing data values were treated "as is" and no imputation was performed.

Reasons for missing data included:
- Outliers removed
- Poor sample specimens
- Samples that were not collected
- Misalignment of peaks or chemical noise in spectra
- Below limit of detection values
- Assay failure or data that failed technical QC
- Those peaks with 50% or more missing values in at least one of the several disease-by-drug groups were excluded from the analysis

### Biomarker Filtering Criteria:

Because many thousand statistical tests were performed, multiple hypothesis testing (i.e., increased chance of false positive results) was an important issue. For example, if one were simply to perform uncorrected independent hypothesis tests at the traditional 5% level, then 10,000 x 0.05 = 500 false positives would be expected if 10,000 biomarkers were tested and none were truly differentially expressed. Therefore, upon completion of the UVDA and MVDA, various statistical cut-offs and biomarker filtering criteria were used to reduce the number of biomarkers and help control the number of false positives. The types of statistical cut-offs are described below and the specific cut-offs used in the various analyses are indicated in their respective sections.

### P-Value Cut-Offs:

For all standard statistical tests, p-values were generated for each biomarker and each treatment comparison. As described in the example above, when performing many hundreds or thousands of statistical tests, an α-level of 0.05 without any adjustment for multiple comparisons may lead to large number of false positives. However, as an extension of the example above, reducing the p-value cut-off from 0.05 to 0.001 reduces the expected false positives from 500 to 10 if 10,000 biomarkers were tested and none were truly differentially expressed. Furthermore, because an FDR value of 0.1 was found, in some cases, to be similar to a p-value cut-off of 0.05, low p-value cut-offs were used to further reduce biomarker lists for biological interpretation. In all cases, p-value cut-offs were used in combination with FDR-values in an attempt to better understand the relationship between p-values and FDR-values, and in controlling for false positives.

### False Discovery Rate:

FDR approaches (Benjamini and Hochberg, J. R. Statist Soc. B. 57:289-300 (1995)) were evaluated to control the average number of false positives in the list of selected biomarkers. Instead of controlling for family-wise error rate, which is very conservative by controlling false positives in all biomarkers, FDR controls the percent of false positives only in the number of selected biomarkers. For integrated biomarker analysis an FDR cut-off of 25% was applied (FDR ≤ 0.25).

### Median Fold Change:

The MFC, which represents the median amount of change in one group compared to the other, was used to help reduce biomarker lists and control for false positives. MFC cut-offs represent a sort of biological filtering criteria and represents the minimal fold-change required to indicate biological relevance. For most platform lists, the cut-off for biological relevance was set at a 1.2-fold change (MFC ≥ 1.2).

### Intensity Cut-Off:

An intensity cut-off was utilized for the transcriptomics data, because intensity levels using MAS 5.0 software are known to be less accurate in the low range of expression. To adjust for this limitation and help control the number of false positives, an intensity criterion was applied. The median scale factor value generated from GeneChip® MOE 430A was calculated. This number was then multiplied by the intensity of an unseated MOE 430A GeneChip® or a factor of 32. The intensity cut-offs for liver, gastroenemius and subcutaneous fat were generated independently and were applied after the univariate biomarker lists were generated. Intensity filtering was applied by eliminating groups in which the median of both comparison groups was below the intensity cut-off value derived for each tissue. In cases where one group was above the limit of detection and the other was below, the biomarker was included and generated.

### Univariate Analysis:

Standard UVDA were performed on all finalized data sets. The UVDA approach examined several main effects (e.g., drug, disease) and interactions (*e.g.,* drug-by-disease). For UVDA, biomarkers were tested individually.

### Analysis of Variance:

The primary UVDA was based on ANOVA. The ANOVA model included main effects (drug and disease), two factor interaction (drug-by-disease), and other experimental blocking factors, e.g., hybridization day for transcriptomics data. For biomarkers measured at the last time-point only: ANOVA was performed using only Day 14 data.

### Results:

The usefulness of Gal-3 and BNP as markers in diabetics treated with a PPAR-γ agonist or a TZD was assessed. Gal-3 expression levels were measured in diabetes model (db/db and HFD) and normal mice. The level of Gal-3 mRNA in adipose tissue was elevated in the disease model animals to levels greater than 4 times the level of Gal-3 mRNA in adipose tissue from normal animals (see Table 1). Likewise, the level of Gal-3 mRNA in muscle tissue was also elevated in the disease model animals to levels greater than twice the level of Gal-3 mRNA in muscle tissue from normal animals (see Table 1). The level of Gal-3 mRNA in liver tissue from the disease model animals was not significantly different, or only modestly significantly different, from the level of Gal-3 in liver tissue from normal animals (see Table 1). Thus, overall, Gal-3 expression appears significantly increased in diabetic animals.

Gal-3 expression levels were also assessed in diseased and healthy mice which had been treated with rosiglitazone, glyburide, or metformin. Treatment of T2DM animals with rosiglitazone causes a significant reduction of Gal-3 mRNA levels in both adipose and muscle tissue (see Table 1). In addition, rosiglitazone modestly elevated Gal-3 mRNA levels in liver tissue in the disease model animals. Thus, while Gal-3 mRNA levels are increased in adipose and muscle tissues from diseased animals, treatment with rosiglitazone reduces Gal-3 mRNA levels in these same tissues.

Neither glyburide nor metformin treatment of the mouse models of T2DM had any significant effect on Gal-3 mRNA levels in adipose tissue, muscle or liver in these animals (see Table 1).

In addition, BNP expression levels were assessed in both untreated animals and in animals being treated with a PPAR-γ agonist or a TZD. The levels of BNP mRNA in adipose, liver and muscle tissue from the diseased animals are not significantly different from the levels of Gal-3 mRNA in the same tissues from normal animals (see Table 2). Similarly, treatment of the disease model animals with rosiglitazone does not have a significant effect on the BNP mRNA levels in the adipose, liver or muscle tissues in those animals, except for a modest elevation in adipose tissue in one animal model (see Table 2). Since BNP levels are not affected in diseased animals or in response to treatment with PPAR-γ agonists or a TZD, comparisons of BNP levels for assessing the presence or progression of heart failure in diabetics can be made to BNP levels in non-diabetics, or to BNP levels in diabetics known to develop heart failure.

**Table 1. Galectin-3 RNA Levels in Mouse T2DM Animal Models**

| **mRNA Type** | **Tissue Type** | **Animal Model & Study #** | **Statistical Comaprison Group A/Group B** | **FDR p-value** | **Raw p-value** | **MFC** | **Direction & Significance** |
|---|---|---|---|---|---|---|---|
| Galectin-3 | Adipose | DB-06 | Disease/Normal Vehicle | <1.00E-8 | 2.38E-26 | 4.77 | Increase Signif |
| Galectin-3 | Adipose | DB-06 | Rosi/Disease | 3.60E-07 | 1.55E-08 | 0.55 | Decrease Signif |
| Galectin-3 | Liver | DB-06 | Disease/Normal Vehicle | 0.807 | 0.596 | 0.99 | Decrease NS |
| Galectin-3 | Liver | DB-06 | Rosi/Disease | 0.135 | 0.026 | 1.33 | Increase Signif |
| Galectin-3 | Muscle | DB-06 | Disease/Normal Vehicle | <1.00E-8 | 5.26E-16 | 2.70 | Increase Signif |
| Galectin-3 | Muscle | DB-06 | Rosi/Disease | 0.974 | 0.101 | 0.87 | Decrease Signif |
| Galectin-3 | Adipose | DB-07 | Disease/Normal Vehicle | <1.00E-8 | 4.32E-17 | 3.83 | Increase Signif |
| Galectin-3 | Adipose | DB-07 | Glyburide/Disease | 0.854 | 0.331 | 1.10 | Increase NS |
| Galectin-3 | Adipose | DB-07 | Metformin/Disease | 0.793 | 0.346 | 1.10 | Increase NS |
| Galectin-3 | Liver | DB-07 | Disease/Normal Vehicle | 0.772 | 0.611 | 0.94 | Decrease NS |
| Galectin-3 | Liver | DB-07 | Glyburide/Disease | 0.999 | 0.922 | 1.01 | Increase NS |
| Galectin-3 | Liver | DB-07 | Metformin/Disease | 0.982 | 0.681 | 1.06 | Increase NS |
| Galectin-3 | Muscle | DB-07 | Disease/Normal Vehicle | <1.00E-8 | 6.76E-17 | 3.28 | Increase Signif |
| Galectin-3 | Muscle | DB-07 | Glyburide/Disease | 0.999 | 0.177 | 1.19 | Increase NS |
| Galectin-3 | Muscle | DB-07 | Metformin/Disease | 0.99 | 0.498 | 0.90 | Decrease NS |
| Galectin-3 | Adipose | HFD-06 | Disease/Normal Vehicle | 0.220 | 0.020 | 1.36 | Increase Signif |
| Galectin-3 | Adipose | HFD-06 | Glyburide/Disease | 0.999 | 0.922 | 1.04 | Increase NS |
| Galectin-3 | Adipose | HFD-06 | Metformin/Disease | 0.877 | 0.401 | 1.08 | Increase NS |
| Galectin-3 | Liver | HFD-06 | Disease/Normal Vehicle | 0.173 | 0.018 | 0.75 | Decrease Signif |
| Galectin-3 | Liver | HFD-06 | G lyburide/Disease | 0.942 | 0.841 | 1.02 | Increase NS |
| Galectin-3 | Liver | HFD-06 | Metformin/Disease | 0.998 | 0.656 | 1.08 | Increase NS |
| Galectin-3 | Muscle | HFD-06 | Disease/Normal Vehicle | 0.894 | 0.458 | 1.27 | Increase NS |
| Galectin-3 | Muscle | HFD-06 | Glyburide/Disease | 0.999 | 0.641 | 0.87 | Decrease NS |
| Galectin-3 | Muscle | HFD-06 | Metformin/Disease | 0.999 | 0.875 | 0.82 | Decrease NS |
| Galectin-3 | Adipose | HFD-07 | Disease/Normal Vehicle | 0.204 | 0.049 | 1.38 | Increase Signif |
| Galectin-3 | Adipose | HFD-07 | Rosi/Disease | 0.024 | 0.004 | 0.64 | Decrease Signif |
| Galectin-3 | Liver | HFD-07 | Disease/Normal Vehicle | 0.017 | 0.002 | 0.57 | Decrease Signif |
| Galectin-3 | Liver | HFD-07 | Rosi/Disease | 0.972 | 0.532 | 1.07 | Increase NS |
| Galectin-3 | Muscle | HFD-07 | Disease/Normal Vehicle | 0.999 | 0.681 | 1.04 | Increase NS |
| Galectin-3 | Muscle | HFD-07 | Rosi/Disease | 0.999 | 0.050 | 0.80 | Decrease NS |

| | | | | | | | |
|---|---|---|---|---|---|---|---|
| Abbreviations for Tables 1 & 2 DB06 = Study 06 with leptin receptor knock out mouse (db/db) model of Type 2 Diabetes DB07 = Study 07 with db/db mouse HFD06 = Study 06 with high fat diet mouse model of Type 2 Diabetes HFD07 = Study 07 with high fat diet mouse FDR = False Discovery Rate (see MFC = Median fold change (ratio of median value of Galectin-3 mRNA in Group A to median value of Galectin-3 mRNA in Group B) Signif= Significant difference between the Group A and Group B median values for Galectin-3 mRNA NS = No Significant Difference based on statistical comparison of Galectin-3 mRNA values in Group A and Group B | | | | | | | |

**Table 2. BNP mRNA Levels in Mouse T2DM Animal Models**

| **mRNA Type** | **Tissue Type** | **Animal Model & Study #** | **Statistical Comaprison Group A/Group B** | **FDR p-value** | **Raw p-value** | **MFC** | **Direction & Significance** |
|---|---|---|---|---|---|---|---|
| BNP | Adipose | DB-06 | Disease/Normal Vehicle | 0.230 | 0.107 | 0.74 | Decrease NS |
| BNP | Adipose | DB-06 | Rosi/Disease | 0.368 | 0.184 | 1.13 | Increase NS |
| BNP | Liver | DB-06 | Disease/Normal Vehicle | 0.350 | 0.128 | 0.67 | Decrease NS |
| BNP | Liver | DB-06 | Rosi/Disease | 0.793 | 0.559 | 1.68 | Increase NS |
| BNP | Muscle | DB-06 | Disease/Normal Vehicle | 0.928 | 0.776 | 1.43 | Increase NS |
| BNP | Muscle | DB-06 | Rosi/Disease | 1.000 | 0.234 | 0.58 | Decrease NS |
| BNP | Adipose | DB-07 | Disease/Nonnal Vehicle | 0.531 | 0.385 | 0.99 | Decrease NS |
| BNP | Adipose | DB-07 | Glyburide/Disease | 0.790 | 0.198 | 1.02 | Increase NS |
| BNP | Adipose | DB-07 | Metformin/Disease | 0.991 | 0.957 | 0.87 | Decrease NS |
| BNP | Liver | DB-07 | Disease/Normal Vehicle | 0.298 | 0.128 | 1.04 | Increase NS |
| BNP | Liver | DB-07 | Glyburide/Disease | 1.000 | 0.406 | 0.85 | Decrease NS |
| BNP | Liver | DB-07 | Metformin/Disease | 0.900 | 0.147 | 0.88 | Decrease NS |
| BNP | Muscle | DB-07 | Disease/Normal Vehicle | 0.474 | 0.212 | 1.46 | Increase NS |
| BNP | Muscle | DB-07 | Glyburide/Disease | 1.000 | 0.462 | 0.83 | Decrease NS |
| BNP | Muscle | DB-07 | Metformin/Disease | 1.000 | 0.464 | 1.09 | Increase NS |
| BNP | Adipose | HFD-06 | Disease/Normal Vehicle | 0.927 | 0.680 | 0.94 | Decrease NS |
| BNP | Adipose | HFD-06 | Glyburide/Disease | 1.000 | 0.673 | 1.16 | Increase NS |
| BNP | Adipose | HFD-06 | Metformin/Disease | 0.638 | 0.005 | 0.67 | Decrease NS |
| BNP | Liver | HFD-06 | Disease/Normal Vehicle | 0.804 | 0.490 | 1.08 | Increase NS |
| BNP | Liver | HFD-06 | Glyburide/Discase | 0.843 | 0.622 | 0.93 | Decrease NS |
| BNP | Liver | HFD-06 | Metformin/Disease | 1.000 | 0.892 | 1.03 | Increase NS |
| BNP | Muscle | HFD-06 | Disease/Normal Vehicle | 0.967 | 0.784 | 0.52 | Decrease NS |
| BNP | Muscle | HFD-06 | Glyburide/Discase | 1.000* | 0.031* | 3.17 | Increase NS |
| BNP | Muscle | HFD-06 | Metformin/Disease | 0.999 | 0.514 | 2.17 | Increase NS |
| BNP | Adipose | HFD-07 | Disease/Normal Vehicle | 0.540 | 0.280 | 0.85 | Decrease NS |
| BNP | Adipose | HFD-07 | Rosi/Disease | 0.103 | 0.033 | 1.29 | Increase Signif |
| BNP | Liver | HFD-07 | Disease/Normal Vehicle | 0.971 | 0.931 | 0.94 | Decrease NS |
| BNP | Liver | HFD-07 | Rosi/Disease | 0.978 | 0.595 | 0.77 | Decrease NS |
| BNP | Muscle | HFD-07 | Disease/Normal Vehicle | 1.000 | 0.366 | 1.23 | Increase NS |
| BNP | Muscle | HFD-07 | Rosi/Disease | 1.000 | 0.480 | 1.40 | Increase NS |
| BNP | Muscle | Clinical | Rosi/Placebo | 0.588 | 0.102 | 0.63 | Decrease NS |

| | | | | | | | |
|---|---|---|---|---|---|---|---|
| See Table 1 for explanation of abbreviations. | | | | | | | |

### Example 3: Assessment of Congestive Heart Risk Using Gal-3 as a Marker

A human diabetes patient undergoing treatment with rosiglitazone is monitored for congestive heart risks by detecting Gal-3 protein levels in the blood. Pretreatment levels of Gal-3 are established for the patient by detecting Gal-3 levels in an initial blood sample. Additional blood samples are obtained from the patient at various times after the initiation of treatment. Gal-3 protein levels in the blood samples are detected using any method well known in the art, such as EL1SA. The Gal-3 protein levels observed, or a trend in Gal-3 protein levels observed over the course of treatment, are compared to a concentration or trend derived from data observed in past diabetes patients treated with rosiglitazone to determine whether the Gal-3 levels in the patient indicate a particular congestive heart risk in the patient. If, for example, Gal-3 levels are increasing during the course of rosiglitazone treatment, the patient is identified as having an elevated congestive heart risk compared to other patients treated with rosiglitazone. Rosiglitazone treatment is discontinued and alternative therapeutic options for treating or managing the patient's diabetes are evaluated.

## Claims

1. A method of assessing congestive heart failure risk in a patient being administered a thiazolidinedione, the method comprising:
detecting the presence or absence of an increasing galectin-3 concentration in a body fluid sample of a patient being treated with a thiazolidinedione,
wherein the presence of an increasing galectin-3 concentration over time is indicative of an increased congestive heart failure risk in the patient.

2. A method according to claim 1, comprising comparing a galectin-3 concentration during a course of treatment with the thiazolidinedione to an earlier galectin-3 concentration during the course of treatment.

3. The method of claim 2, comprising comparing galectin-3 concentrations at several times during the course of treatment with the thiazolidinedione, thereby to enable development of a history of said concentrations.

4. A method of assessing a candidate for treatment with a thiazolidinedione, the method comprising:
measuring a galectin-3 concentration in a body fluid sample of a patient having a condition treatable with a thiazolidinedione;
comparing the measured galectin-3 concentration to a reference galectin-3 concentration, wherein the reference galectin-3 concentration is derived from concentrations of galectin-3 in other patients having the condition and is indicative of congestive heart failure risk in patients having the condition; and
recommending that administration of the thiazolidinedione be restricted or discontinued if the measured galectin-3 concentration exceeds the reference galectin-3 concentration.

5. The method of claim 4, wherein the method comprises refusing administration of the thiazolidinedione if the measured galectin-3 concentration exceeds the reference galectin-3 concentration.

6. A method according to any one of claims 1 to 5, wherein the thiazolidinedione is rosiglitazone.

7. A method according to any one of claims 1 to 6, wherein the body fluid sample comprises blood, serum or plasma.

8. A method according to any one of claims 1 to 7, wherein the patient is a diabetes patient.

9. A method of assessing congestive heart failure risk in a patient being administered a peroxisome-proliferator-activator-receptor-y agonist, the method comprising:
detecting the presence or absence of an increasing galectin-3 concentration in a body fluid sample of a patient being treated with a peroxisome-proliferator-activator-receptor-γ agonist,
wherein the presence of an increasing galectin-3 concentration over time is indicative of an increased congestive heart failure risk in the patient.

10. A method according to claim 9, comprising comparing a galectin-3 concentration during a course of treatment with the peroxisome-proliferator-activator-receptor-y agonist to an earlier galectin-3 concentration during the course of treatment.

11. The method of claim 10, comprising comparing galectin-3 concentrations at several times during the course of treatment with the peroxisome-proliferator-activator-receptor-γ agonist, thereby to enable development of a history of said concentrations.

12. A method according to claim 9, wherein the peroxisome-proliferator-activator-receptor-γ agonist is a thiazolidinedione.

13. A method according to claim 12, wherein the thiazolidinedione is rosiglitazone.

14. A method according to any of claims 9 to 13, wherein the body fluid sample comprises blood, serum or plasma.

15. A method according to any one of claims 9 to 14, wherein the patient is a diabetes patient.

## Patentansprüche

1. Verfahren zum Feststellen eines Herzinsuffizienzrisikos bei einem Patienten, dem Thiazolidinedion verabreicht wurde, wobei das Verfahren folgendes umfasst:
Nachweisen der Anwesenheit oder Abwesenheit einer zunehmenden Galectin-3-Konzentration in einer Körperflüssigkeitsprobe eines Patienten, der mit einem Thiazolidinedion behandelt wird.
wobei das Vorhandensein einer erhöhten Galectin-3-Konzentration über die Zeit auf ein verstärktes Herzinsuffizienz-Risiko bei dem Patienten hinweist.

2. Verfahren nach Anspruch 1, das den Vergleich einer Galectin-3-Konzentration während einer Behandlung mit dem Thiazolidinedion mit einer früheren Galectin-3-Konzentration während der Behandlung umfasst.

3. Verfahren nach Anspruch 2, das den Vergleich der Galectin-3-Kontentrationen zu verschiedenen Zeitpunkten während der Behandlung mit dem Thiazolidinedion umfasst, wodurch die Erstellung eines Zeitverlaufs der Konzentrationen ermöglicht wird.

4. Verfahren zum Feststellen eines Kandidaten für die Behandlung mit einem Thiazolidinedion, wobei das Verfahren folgendes umfasst:
Messen einer Galectin-3-Konzentration in einer Körperflüssigkeitsprobe eines Patienten, der einen mit einem Thiazolidinedion behandelbaren Zustand aufweist;
Vergleichen von der gemessenen Galectin-3-Konzentration mit einer Galectin-3-Referenzkonzentration, wobei die Galectin-3-Referenzkonzentration aus den Konzentrationen von Galectin-3 in anderen Patienten abgeleitet ist, die den Zustand aufweisen und deren Konzentration auf das Risiko der Herzinsuffizienz in Patienten hinweist, die diesen Zustand aufweisen; und
Empfehlen, dass die Verabreichung des Thiazolidinedions eingeschränkt oder abgesetzt wird, wenn die gemessene Galectin-3-Konzentration die Galectin-3-Referenzkonzentration übersteigt.

5. Verfahren nach Anspruch 4, wobei das Verfahren das Verweigern der Verabreichung von dem Thiazolidinedion umfasst, wenn die gemessene Galectin-3-Konzentration die Galectin-3-Referenz-konzentration übersteigt.

6. Verfahren nach einem der Ansprüche 1 bis 5, wobei das Thiazolidinedion Rosiglitazon ist.

7. Verfahren nach einem der Ansprüche 1 bis 6, wobei die Körperflüssigkeitsprobe Blut, Serum oder Plasma umfasst.

8. Verfahren nach einem der Ansprüche 1 bis 7, wobei der Patient ein Diabetespatient ist.

9. Verfahren zum Feststellen eines Herzinsuffizienzrisikos bei einem Patienten, dem ein Agonist des Peroxisomen-Proliferator-Aktivator-Rezeptor-γ verabreicht wurde, wobei das Verfahren folgendes umfasst:
Nachweisen der Anwesenheit oder Abwesenheit einer zunehmenden Galectin-3-Konzentration in einer Körperflüssigkeitsprobe eines Patienten, der mit einem Agonisten des Peroxisomen-Proliferator-Aktivator-Rezeptor-γ behandelt wird,
wobei das Vorhandensein einer erhöhten Galectin-3-Konzentration über die Zeit auf ein verstärktes Herzinsuffizienz-Risiko bei dem Patienten hinweist.

10. Verfahren nach Anspruch 9, das den Vergleich einer Galectin-3-Konzentration während einer Behandlung mit dem Agonisten des Peroxisomen-Proliferator-Aktivator-Rezeptor-γ mit einer früheren Galectin-3-Konzentration während der Behandlung umfasst.

11. Verfahren nach Anspruch 10, das den Vergleich der Galectin-3-Kontentrationen zu verschiedenen Zeitpunkten während der Behandlung mit dem Agonisten des Peroxisomen-Proliferator-Aktivator-Rezeptor-γ umfasst, wodurch die Erstellung eines Zeitverlaufs der Konzentrationen ermöglicht wird.

12. Verfahren nach Anspruch 9, wobei der Agonist des Peroxisomen-Proliferator-Aktivator-Rezeptor-γ ein Thiazolidonedion ist.

13. Verfahren nach Anspruch 12, wobei das Thiazolidinedion Rosiglitazon ist.

14. Verfahren nach einem der Ansprüche 9 bis 13, wobei die Körperflüssigkeitsprobe Blut, Serum oder Plasma umfasst.

15. Verfahren nach einem der Ansprüche 9 bis 14, wobei der Patient ein Diabetespatient ist.

## Revendications

1. Procédé d'évaluation du risque d'insuffisance cardiaque congestive chez un patient auquel est administrée une thiazolidinedione, le procédé comprenant :
la détection de la présence ou de l'absence d'une concentration croissante de galectine-3 dans un échantillon de fluide corporel d'un patient traité avec une thiazolidinedione,
dans lequel la présence d'une concentration croissante de galectine-3 au fil du temps est indicative d'un risque d'insuffisance cardiaque congestive accrue pour le patient.

2. Procédé selon la revendication 1, comprenant la comparaison d'une concentration de galectine-3 au cours d'un traitement avec la thiazolidinedione à une concentration antérieure de galectine-3 au cours du traitement.

3. Procédé selon la revendication 2, comprenant la comparaison des concentrations de galectine-3, à plusieurs moments pendant la durée du traitement avec la thiazolidinedione, afin de permettre ainsi le développement d'un historique desdites concentrations.

4. Procédé d'évaluation d'un candidat au traitement avec une thiazolidinedione, le procédé comprenant :
la mesure d'une concentration de galectine-3 dans un échantillon de fluide corporel d'un patient présentant une condition pouvant être traitée avec une thiazolidinedione ;
la comparaison de la concentration de galectine-3 mesurée à une concentration de galectine-3 de référence, dans laquelle la concentration de galectine-3 de référence est tirée de concentrations de galectine-3 sur d'autres patients ayant la même condition et est indicative d'un risque d'insuffisance cardiaque congestive chez des patients ayant la même condition ; et
la recommandation que l'administration de la thiazolidinedione soit limitée ou interrompue si la concentration mesurée de galectine-3 dépasse la concentration de référence de galectine-3.

5. Procédé selon la revendication 4, dans lequel le procédé comprend le refus d'administrer la thiazolidinedione si la concentration mesurée de galectine-3 dépasse la concentration de référence de galectine-3.

6. Procédé selon l'une quelconque des revendications 1 à 5, dans lequel la thiazolidinedione est la rosiglitazone.

7. Procédé selon l'une quelconque des revendications 1 à 6, dans lequel l'échantillon de fluide corporel comprend le sang, le sérum ou le plasma.

8. Procédé selon l'une quelconque des revendication 1 à 7, dans lequel le patient est un patient diabétique.

9. Procédé d'évaluation d'un risque d'insuffisance cardiaque congestive chez un patient auquel a été administré un agoniste de proliférateur-activateur-récepteur-γ de peroxisome, le procédé comprenant :
la détection de la présence ou de l'absence d'une concentration croissante de galectine-3 dans un échantillon de fluide corporel d'un patient traité avec un agoniste de proliférateur-activateur-récepteur-γ de peroxisome,
dans lequel la présence d'une concentration croissante de galectine-3 au fil du temps est indicative d'un risque d'insuffisance cardiaque accru chez le patient.

10. Procédé selon la revendication 9, comprenant la comparaison d'une concentration de galectine-3 pendant un traitement avec l'agoniste de proliférateur-activateur-récepteur-γ de peroxisome, à une concentration de galectine-3 antérieure pendant la durée du traitement.

11. Procédé selon la revendication 10, comprenant la comparaison de concentrations de galectine-3, à plusieurs moments au cours du traitement avec l'agoniste de proliférateur-activateur-récepteur-γ de peroxisome, afin de permettre ainsi le développement d'un historique desdites concentrations.

12. Procédé selon la revendication 9, dans lequel l'agoniste de proliférateur-activateur-récepteur-γ de peroxisome est une thiazolidinedione.

13. Procédé selon la revendication 12, dans lequel la thiazolidinedione est la rosiglitazone.

14. Procédé selon l'une quelconque des revendications 9 à 13, dans lequel l'échantillon de fluide corporel comprend le sang, le sérum ou le plasma.

15. Procédé selon l'une quelconque des revendications 9 à 14, dans lequel le patient est un patient diabétique.
